# EUROPEAN PATENT SPECIFICATION

(11) **EP 0 498 905 B1**
(45) Date of publication and mention of the grant of the patent: **23.10.1996**
(21) Application number: 91101938.8
(22) Date of filing: 12.02.1991
(51) Int. Cl.: C07K 14/16, A61K 39/21

(54) **Conformational epitopes of human immunodeficiency virus envelope glycoprotein gp120**
Konformationsepitope des humanen Immundefizienzvirus-Hüllprotein-gp120
Epitopes conformationnelles de la glycoprotéine gp120 d'enveloppe du virus d'immunodéficience humain

(43) Date of publication of application: 19.08.1992
(73) Proprietor: New York Blood Center, Inc., New York, New York 10021 (US)
(72) Inventor: Neurath, Alexander Robert, New York, New York 10021 (US)
(74) Representative: Cohausz & Florack Patentanwälte

(56) References cited:
- EP-A- 0 330 359
- WO-A-90/00901
- IMMUNOLOGY TODAY, vol. 11, no. 11, November 1990, pages 418-425, Elsevier Science Publishers Ltd, Cambridge, GB; J. A. HABESHAW et al.: "AIDS pathogenesis: HIV envelope and its interaction with cell proteins"
- THE JOURNAL OF VIROLOGY, vol. 65, no. 1, January 1991, pages 489-493, American Society for Microbiology; D. D. HO et al.: "Conformational epitope on gp120 important in CD4 binding and human immunodeficiency virus type 1 neutralization identified by a human monoclonal antibody"

## Description

### BACKGROUND OF THE INVENTION

### Field of the Invention

The present invention concerns a portion of a conformational epitope of human immunodeficiency virus (HIV-1) envelope glycoprotein gp120. More particularly, the present invention relates to sequences within the region (113-142) of HIV-1 gp120.

### Description of the Related Art

Virus neutralizing and protective antibodies against HIV-1 are primarily directed against a loop comprising residues (303-338) (residue numbering according to Ratner, L., Haseltine, W., Patarca, R., Livak, K.J., Starchich, B., Josephs, S.F., Duran, E.R., Rafalski, J.A., Whitehorn, E.A., Baumeister, K., Ivanoff, L., Peteway, S.R., Jr., Pearson, M.L., Lautenberger, J.A., Papas, T.S., Ghrayeb, J., Chang, N.T., Gallo, R.C. and Wong-Staal, F., "Complete Nucloetide Sequence of The AIDS Virus, HTLV-III, Nature, 313:277-284, 1985) (Goudsmit, J., (1988), "Immunodominant B-Cell Epitopes of the HIV-1 Envelope Recognized by Infected and Immunized Hosts., AIDS, 2, S41-S45; Goudsmit, J. Debouck, C., Meloen, R.H., Smit, L., Bakker, M., Asher, D.M., Wolff, A.V., Gibbs, Jr., C.J. and Gajdusek, D.C., (1988), "Human Immunodeficiency Virus Type 1 Neutralization Epitope With Conserved Architecture Elicits Early Type-Specific Antibodies in Experimentally Infected Chimpanzees", Proc. Natl. Acad. Sci. USA, 85, 4478-4482; Matsushita, S., Robert-Guroff, M., Rusche, J., Koito, A., Hattori, T., Hoshino, H., Javaherian, K., Takatsuki, K. and Putney, S.D., (1988), "Characterization of a Human Immunodeficiency Virus Neutralizing Monoclonal Antibody and Mapping of the Neutralizing Epitope", J. Virol., 62, 2107-2114; Palker, T.J., Clark, M.E., Langlois, A.J., Matthews, T.J., Weinhold, K.J., Randall, R.R., Bolognesi, D.P. and Haynes, B.F., (1988), "Type-Specific Neutralization of the Human Immunodeficiency Virus With Antibodies to Env-Encoded Synthetic Peptides", Proc. Natl. Acada. Sci. USA, 85, 1932-1936; Rusche, J.R., Javaherian, K., McDanal, C., Petro, J., Lynn, D.L., Grimaila, R., Langlois, A., Gallo, R.C., Arthur, L.O., Fischinger, P.J., Bolognesi, D.P., Putney, S.D. and Matthews, T.J., (1988), "Antibodies that Inhibit Fusion of Human Immunodeficiency Virus-Infected Cells Bind a 24-Amino Acid Sequence of the Viral Envelope, gp120", Proc. Natl. Acad. Sci. USA, 85, 3198-3202; Neurath, A.R., Strick, N. and Lee, E.Y.S., (1990), "B-Cell Epitope Mapping of Human Immunodeficiency Virus (HIV-1) Envelope Glycoproteins With Long (19 to 36-residue) Synthetic Peptides", J. Gen. Virol., in press; and Javaherian, K., Langluis, A.J., McDanal, C., Ross, K.L., Eckler, L.I., Jellis, C.L., Profy, A.T., Rusche, J.A., Bolognesi, D.P., Putney, S.D. and Matthews, T.J., "Principal Neutralizing Domain of Human Immunodeficiency Virus Type 1 Envelope Protein", Proc. Natl. Acad. Sci., USA, 86:6768-6772 (1989).

This sequence, (303-338), is part of a hypervariable V3 region differing greatly among distinct HIV-1 isolates (Myers, G., Rabson, A.B., Josephs, S.F., Smith, T.F. and Wong-Staal, F., (1988), "Human Retroviruses and AIDS 1988: A Compilation and Analysis of Nucleic Acid and Amino Acid Sequences", Los Alamos National Laboratory, Los Alamos, New Mexico). Therefore, virus-neutralizing antibodies elicited by this region of HIV-1 are subtype specific (Neurath, A.R. and Strick, N. (1990), "Confronting the Hypervariability of an Immunodominant Epitope Eliciting Virus Neutralizing Antibodies From the Envelope Glycoprotein of the Human Immunodeficiency Virus Type 1 (HIV-1)", Mol. Immunol., submitted for publication).

The discovery of neutralization escape mutants, having hypervariable V3 loop sequences identical to HIV-1 isolates which can be neutralized (Willey, R.L., Ross, E.K., Buckler-White, A.J., Theodore, T.S. and Martin, M.A., (1989), "Functional Interaction of Constant and Variable Domains of Human Immunodeficiency Virus Type 1 gp120", J. Virol., 63, 3595-3600; Nara, P. Dunlop, N., Hatch. W., Waters, D., Merges, M., Smit, L., Gallo, R. and Goudsmit, J., (1990), "Sequence Variation of the HIV-1 Neutralization Determinant, in Vaccines 90 (Edited by R.A. Lerner, H. Ginsburg, R.M. Chanock and F. Brown), Cold Spring Harbor Laboratory, New York, (in press)), indicates that amino acid replacements outside the hypervariable loop can dramatically affect the recognition by antibodies of epitopes within the hypervariable loop. These results also suggest that virus neutralizing and protective antibodies are directed against conformationally dependent epitopes, only a part of which corresponds to sequences within the region (303-338) of HIV-1 gp120. Delineation of other sequences comprising this epitope is essential for understanding the process of virus-neutralization and for developing protective immunogens against HIV-1 infection.

### DEFINITIONS

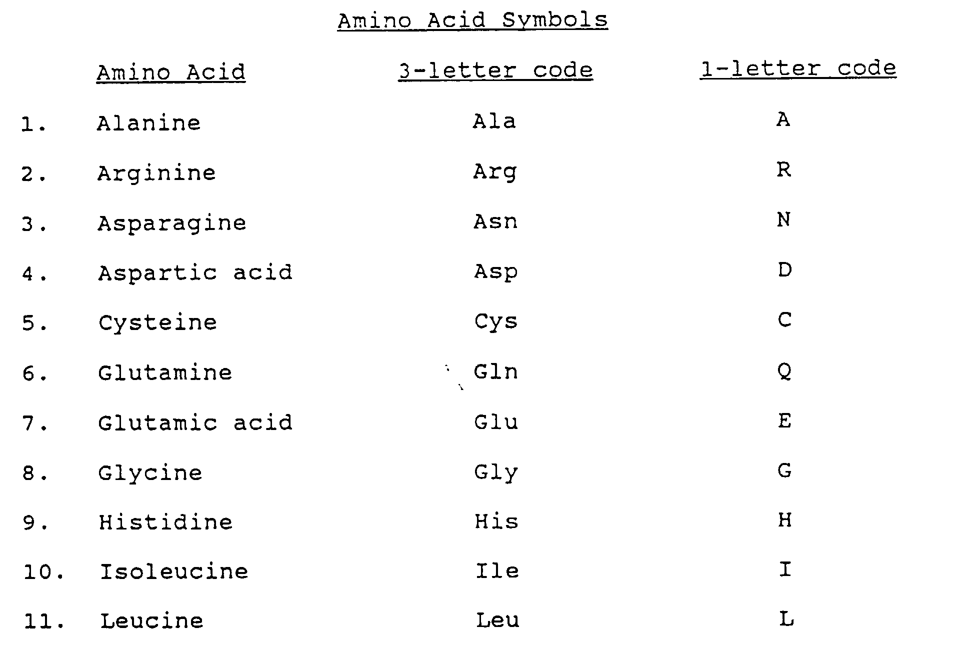

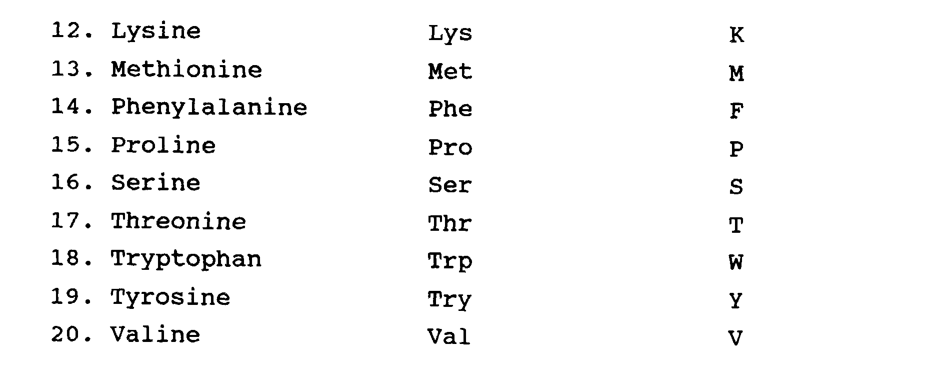

WO-A-90/00901 suggests a peptide antigen which stimulates in individuals of a plurality of diverse major histo-compatibility types, proliferation of T-cells capable of recognizing cells expressing HIV envelope protein. Such peptides are peptides HP6-8 which span amino acid residues 112 to 134 (HEDIISLWDQSLKPCVKLTPLCV).

The above objects and other objects, aims and advantages are satisfied by the present invention. The present invention concerns a polypeptide comprising
(a) the amino acid sequence: 
126 137
EDIISLWDQSLKPCVKLTPLCVSLKCTDLK
113 133 142
 of the envelope glycoprotein gp120 of the BH-10 isolate of the human immunodeficiency virus or an analogous amino acid sequence from a human immunodeficiency virus gp120 isolate other than BH-10;
   linked by an amino acid spacer sequence to:
(b) an amino acid sequence comprising 5 to 36 amino acids in the amino acid sequence: 
CTRPNNNTRKSIRIQRGPGRAFVTIGKIGNMRQAHC
303                                                                                    338
 of the envelope glycoprotein gp120 of the BH-10 isolate of the human immunodeficiency virus or an analogous amino acid sequence from a human immunodeficiency virus gp120 isolate other than BH-10;
   said polypeptide not comprising more than 302 amino acids and said polypeptide not comprising a sequence identical with that of the entire natural gp120.

According to an embodiment, the polypeptide comprises the amino acid sequence 113-338 of the envelope glycoprotein gp120 of the BH-10 isolate of the human immunodeficiency virus or an analogous amino acid sequence from a human immunodeficiency virus gp120 isolate other than BH-10.

Figs. 1a, 1b and 1c are three graphs depicting the effect of monoclonal antibody NEA-9305 on the attachment to HIV-1 gp 160 of distinct rabbit anti-peptide antisera. Fig. la is for anti-peptide (113-142)_{aba133} anitserum. Fig. lb is for anti-peptide (306-338) antiserum. Fig. lc is for antipeptide (303-338) antiserum.

Figs. 2a, 2b and 2c are three graphs depicting the effect of goat anti-SP10 on the attachment to HIV-1 gp 160 of distinct rabbit anti-peptide antisera. Fig. 2a is for anti-(113-142)_{Aba133}. Fig. 2b is for anti-(306-338). Fig. 2c is for anti-(303-338).

The present invention concerns a polypeptide of a conformational epitope of human immunodeficiency virus (HIV-1) envelope glycoprotein gp120 comprising the sequence (113-142) 
126          137
EDIISLWDQSLKPCVKLTPLCVSLKCTDLK
113                133       142
 of the BH-10 subtype, not comprising more than 302 amino acids, and analogous sequences from other HIV-1 subtypes, some of which are shown as follows below:

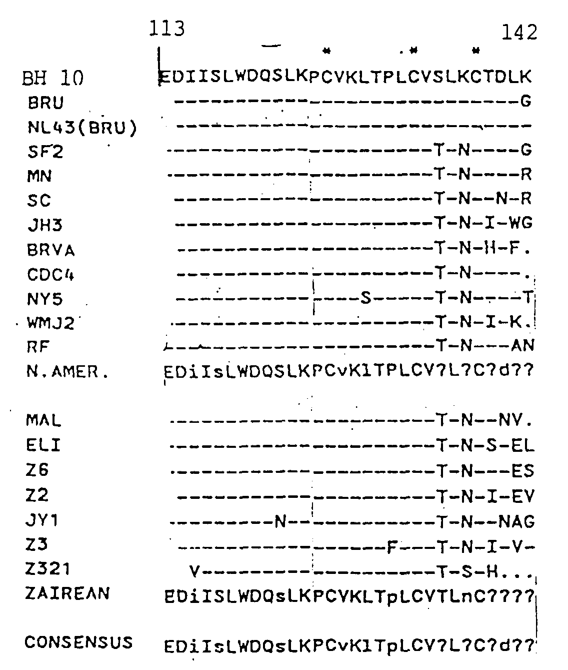

In an embodiment of the invention, the cysteine ("C") at position 133 is replaced by γ-aminobutyric acid (Aba) at position 133.

In an embodiment of the invention, the full length sequence of the thirty amino acids of sequence (113-142) is utilized and in another embodiment of the invention, such full length sequence (113-142) is employed and the cysteine at position 133 is replaced by γ-aminobutyric acid.

In a further embodiment of the present invention, the epitope comprises the sequence (126-137) CVKLTPLCVSLK.

Generally the peptide according to the invention has no more than 302 amino acids, preferably no more than 100 amino acids.

The inventive peptides can be either synthetic peptides (peptides produced by assembling individual amino acids by chemical means or by using expression vectors (DNA route) or peptides derived from natural sources. In the formation of a peptide derived from natural sources, a protein containing the required amino acid sequence is subjected to selective proteolysis such as by splitting the protein with chemical reagents or using enzymes.

The length of the individual amino acid sequence would depend on the method of producing the sequence. If the sequence is made by assembling individual amino acids by chemical means, then the sequence length would generally not exceed 50 amino acids. If the peptide is obtained from a DNA route, the chain length could be longer, for example, 100 or more amino acids. It is, however, normally shorter, and considerably shorter than the naturally occurring human immunodeficiency virus envelope gp120.

Where, however, the amino acid sequence is part of a long chain, such as when there are more than one sequence of amino acids, the chain can contain residues of various moieties, for example, segments of polyamino acids or polysaccharides.

In an embodiment according to the invention, one or more peptides can be employed in a composition with at least 5 to 36 consecutive amino acids in the BH-10 HIV-1 sequence (303-338): 
CTRPNNNTRKSIRIQRGPGRAFVTIGKIGNMRQAHC
303                                338,
 or analogous sequences corresponding to HIV-1 subtypes other than BH-10, some of which are listed hereinbelow as follows:

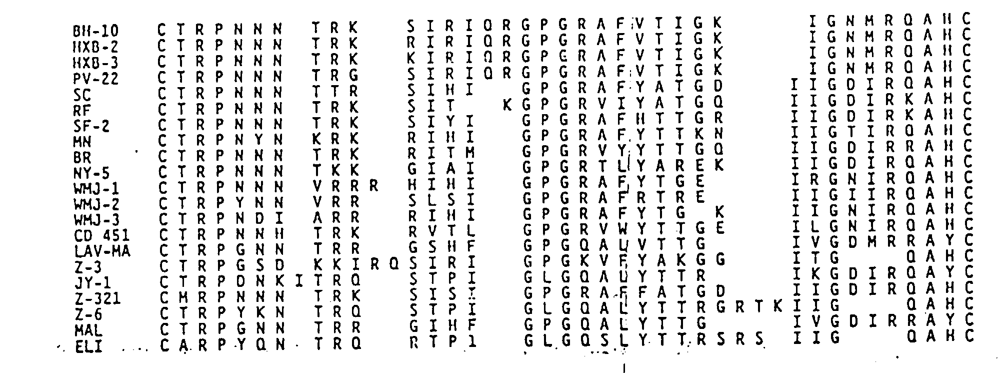

In a further embodiment, an inventive peptide with γ -aminobutyric acid, instead of cysteine, at position 133 can be employed in a composition with at least six consecutive amino acids in the HIV-1 sequence (303-338).

Sequences comprising (113-142) can be combined with sequences comprising (303-338) by using a spacer, e.g., spacer amino acids, by using a common carrier, by using separate carriers, or by having an amino acid sequence spanning both (113-142) and (303-338).

Anti-HIV-1 effective amounts of (1) one or more inventive peptides or (2) the aforesaid compositions comprising (a) one or more inventive peptides and (b) one or more amino acid sequences in the HIV-1 sequence (303-338), can be admixed with a physiologically acceptable diluent, e.g., phosphate buffered saline, to form anti-HIV-1 vaccines.

The peptides (sequences) according to the invention can be utilized to induce the production of neutralizing antibodies to HIV-1 by administering to an animal, for example, a warm blooded animal, e.g., a human, an anti-HIV-1 effective amount of an inventive peptide (sequence).

The peptides according to the invention can also be employed in HIV-1 diagnostics, for example, to detect antibodies to HIV-1.

In forming a synthetic vaccine according to the present invention, it is preferred to insure that the amino acid has a steric configuration to be recognized by antibody to HIV-1. To this end, the given chain of amino acids may have bonded thereto as part of the amino acid chain, one or more additional amino acids on either, or both ends thereof. These additional amino acids can serve as auxiliary amino acids to enhance the stabilization of the amino acid chain so that it is readily recognized by antibody to HIV-1. The additional amino acids can be the same amino acids in the same sequence as they occur in the natural protein, or other amino acids may be employed.

A carrier may be provided for the synthetic peptide of the invention. It should be understood, however, that a carrier may not be required to practice the present invention, i.e., a carrier may not be required to produce an immune response.

The "carrier" is simply a physiologically acceptable mass to which the peptide is attached and which is expected to enhance the immune response. A carrier can comprise simply a chain of amino acids or other moieties. It is preferred that alternative carriers comprise some substance, animal, vegetable or mineral, which is physiologically acceptable and functions to present the peptide so that it is recognized by the immune system of a host and stimulates a satisfactory immunological response. Thus, a wide variety of carriers are contemplated, and these include materials which are inert, which have biological activity and/or promote an immunological response. Examples of protein carriers include tetanus toxoid and keyhole lympet hemocyanin.

The peptides of the invention can be linked to carriers by crosslinking agents employed to interconnect a plurality of synthetic peptide containing chains.. Crosslinking agents which have as their functional group an aldehyde (such as glutaraldehyde), carboxyl, amine, amido, imido or azidophenyl group are preferred.

A carrier for the peptides of the present invention can also be a lipid vesicle. Lipid vesicles can be formed by sonicating a lipid in an aqueous medium, by resuspension of dried lipid layers in a buffer or by dialysis of lipids dissolved in an organic solvent against a buffer of choice.

Chemical synthesis of peptides is described in the following publications: S.B.H. Kent, Biomedical Polymers, eds. Goldberg, E.P. and Nakajima, A. (Academic Press, New York), 213-242, (1980); A.R. Mitchell, S.B.H. Kent, M. Engelhard, and R.B. Merrifield, J. Org. Chem., 43, 2845-2852, (1978); J.P. Tam, T.-W. Wong, M. Riemen, F.-S. Tjoeng and R.B. Merrifield, Tet. Letters, 4033-4036, (1979); S. Mojsov, A.R. Mitchell and R.B. Merrifield, J. Org. Chem., 45, 555-560, (1980); J.P. Tam, R.D. DiMarchi and R.B. Merrifield, Tet. Letters, 2851-2854, (1981) and S.B.H. Kent, M. Riemen, M. Le Doux and R.B. Merrifield, Proceedings of the IV International Symposium on Methods of Protein Sequence Analysis, (Brookhaven Press, Brookhaven, N.Y.), 1981.

In the Merrifield solid phase chemical synthesis procedure, the appropriate sequence of L-amino acids is built up from the carboxyl terminal amino acid to the amino terminal amino acid. Starting with the appropriate carboxyl terminal amino acid attached to a polystyrene (or other appropriate) resin via chemical linkage to a chloromethyl group, benzhydrylamine group, or other reactive group of the resin, amino acids are added one by one using the following procedure. The peptide resin is:
(a) washed with methylene chloride;
(b) neutralized by mixing for 10 minutes at room temperature with 5% (v/v) diisopropylethylamine (or other hindered base) in methylene chloride;
(c) washed with methylene chloride;
(d) an amount of amino acid equal to six times the molar amount of the growing peptide chain is activated by combining it with one-half as many moles of a carbodiimide (e.g., dicyclohexylcarbodiimide, or diisopropylcarbodiimide) for ten minutes at 0°C, to form the symmetric anhydride of the amino acid. The amino acid used should be provided originally as the N-alpha-tert.butyloxycarbonyl derivative, with side chains protected with benzyl esters (e.g., aspartic or glutamic acids), benzyl ethers (e.g., serine, threonine, cysteine or tyrosine), benzyloxycarbonyl groups (e.g., lysine) or other protecting groups commonly used in peptide synthesis;
(e) the activated amino acid is reacted with the peptide-resin for two hours at room temperature, resulting in addition of the new amino acid to the end of the growing peptide chain;
(f) the peptide-resin is washed with methylene chloride;
(g) the N-alpha-(tert. butyloxycarbonyl) group is removed from the most recently added amino acid by reacting with 30 to 65%, preferably 50% (v/v) trifluoroacetic acid in methylene chloride for 10 to 30 minutes at room temperature;
(h) the peptide-resin is washed with methylene chloride; and
(i) steps (a) through (h) are repeated until the required peptide sequence has been constructed.

The peptide is then removed from the resin and simultaneously the side-chain protecting groups are removed, by reaction with anhydrous hydrofluoric acid containing 10% v/v of anisole or other suitable (aromatic) scavenger. Subsequently, the peptide can be purified by gel filtration, ion exchange, high pressure liquid chromatography, or other suitable means.

In some cases, chemical synthesis can be carried out without the solid phase resin, in which case the synthetic reactions are performed entirely in solution. The reactions are similar and well known in the art, and the final product is essentially identical.

The inventive peptide can also be isolated from natural sources. If sufficient quantities of the whole protein antigen are available, a limited portion of the molecule, bearing the desired sequence of amino acids may be excised by any of the following procedures:
(a) Digestion of the protein by proteolytic enzymes, especially those enzymes whose substrate specificity results in cleavage of the protein at sites immediately adjacent to the desired sequence of amino acids;
(b) Cleavage of the protein by chemical means. Particular bonds between amino acids can be cleaved by reaction with specific reagents. Examples include: bonds involving methionine are cleaved by cyanogen bromide; asparaginylglycine bonds are cleaved by hydroxylamine;
(c) A combination of proteolytic and chemical cleavages.

It is also possible to clone a small portion of the DNA, either from natural sources or prepared by synthetic procedures, or by methods involving a combination thereof, that codes for the desired sequence of amino acids, resulting in the production of the peptide by bacteria, or other cells.

One can also form chains containing a plurality of amino acid sequences by the following technique: An aqueous solution of a peptide or peptides is mixed with a water-soluble carbodiimide (e.g., ethyl- dimethylaminopropylcarbodiimide). This results in polymerization of the peptide(s); depending on the use of the side chain blocking groups mentioned above, either straight chain or branched polymers of the peptide can be made.

If desired a peptide of the present invention can have bonded thereto a chain of any of the following moieties: polypeptide, polyamino acid, polysaccharide, polyamide or polyacrylamide which can serve as a stabilizing chain or as a bridge between amino acids of the individual chains. Such chains are available commercially or, in the case of polyamino acids, are formed by a process which comprises: mixing a solution of the desired amino acid sequence with a solution of the N-carboxylanhydride of the amino acid and allowing a base-catalyzed polymerization to occur, which is initiated by the amine groups of the peptide.

The present invention is also directed to diagnostic tests for direct detection of HIV-1 antigens and HIV-1 antibodies using conventional techniques such as radioimmunoassay (RIA), enzyme-linked immunosorbent assay (ELISA) or fluorescence techniques.

The labelling ("marking") of one of the reaction components in a diagnostic assay can be brought about by use of a "marker", "marker substance" or "label" such as by incorporation of a radioactive atom or group, or by coupling of an immunologic component to an enzyme, a dyestuff, e.g., chromophobic moiety, or a fluorescent group.

The immunologic components concerned are preferably labelled by coupling to an enzyme, since the estimation of this is much simpler than, for example, the estimation of radioactivity, for which special apparatus is necessary.

The enzymes used are preferably those which can be colorimetrically, spectrophotometrically, or fluorimetrically determined. Non-limiting examples of enzymes for use in diagnostic assays include enzymes from the group of oxidoreductases, such as catalase, peroxidase, glucose oxidase, beta-glucuronidase, beta-D-glucosidase, beta-D-galactosidase, urease and galactose oxidase. Also the following enzymes can be employed: alkaline phosphatase and beta-lactamase.

The coupling of the enzyme and the immunological component can be brought about in a known way, for example, by the formation of an amide linkage by methods known form peptide chemistry.

Labelling with a radioactive isotope can also be performed in a known way. Isotopes useful for labelling are predominantly I¹²⁵, I¹³¹, C¹⁴ and H³.

In general, all known immunoassays can be performed. These immunoassays include all those disclosed by Langone and Van Vunakis, Methods of Enzymology, Academic Press, Volumes 70, 73 and 74. Those assays disclosed in the disclosures of the following U.S. Patents: 4,459,359; 4,343,896; 4,331,761; 4,292,403; 4,228,240; 4,157,280; 4,152,411; 4,169,012; 4,016,043; 3,839,153; 3,654,090 and Re 31,006 and volumes 70, 73 and 74 of Methods of Enzymology are incorporated herein by reference.

The active component of a vaccine, i.e., comprising an inventive peptide, can be employed with a physiologically acceptable diluent (medium), e.g., phosphate buffered saline. Generally speaking, the peptide concentration in a physiologically acceptable medium will be between approximately less than 1 milligram and more than 10 micrograms per dose.

The vaccine can be administered to a warm blooded animal, e.g., a human, by, for example, subcutaneous, intradermal or intramuscular injection. While the preferred route would depend upon the particular vaccine, it is believed that intramuscular injection will be generally suitable. Frequency of administration will vary depending upon the vaccine. Generally speaking, the vaccine will be administered in two doses about one month apart followed by a booster at six months to one year after primary immunization. The subsequent doses or the booster will depend on the level of antibody in the blood as a result of the initial immunization, and in certain instances may be unnecessary.

Adjuvants may also be employed in the vaccine.

In order to more fully illustrate the nature of the invention and the manner of practicing the same, the following non-limiting examples are presented:

### EXAMPLES

### Example 1

The strategy for establishing the proximity within the three-dimensional structure of gp120 of amino acid residues non-adjacent in the primary amino acid sequence was as follows: Antisera to peptides from nearly the entire sequence of gp120 were prepared, including antisera to sequences from the V3 hypervariable loop (residues 303-338); Modrow, S., Hahn, B.H., Shaw, G.M., Gallo, R.C., Wong-Staal, F. and Wolf, H., (1987), "Computer-assisted Analysis of Envelope Protein Sequences of Seven Human Immunodeficiency Virus Isolates: Prediction of Antigenic Epitopes in Conserved and Variable Regions", J. Virol., 2:570-578). It was expected that antibodies directed against sequences proximal in the three-dimensional structure of HIV-1 gp120, but nonadjacent in the primary sequence of gp120 to residues (303-338) would at least partly inhibit the attachment of anti-(303-308) [anti-(306-338)] antibodies to the HIV envelope glycoproteins gp120 or gp160. Due to technical difficulties in detecting competition between antibodies of distinct specificities derived from the same animal species (rabbits), the competition of mouse monoclonal antibodies (McAbs) directed against the V3 hypervariable loop [i.e., the sequence (315-329)] with the attachment of distinct anti-peptide antisera (Table 1) was studied.

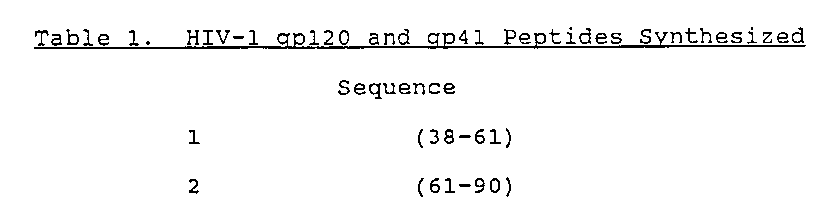

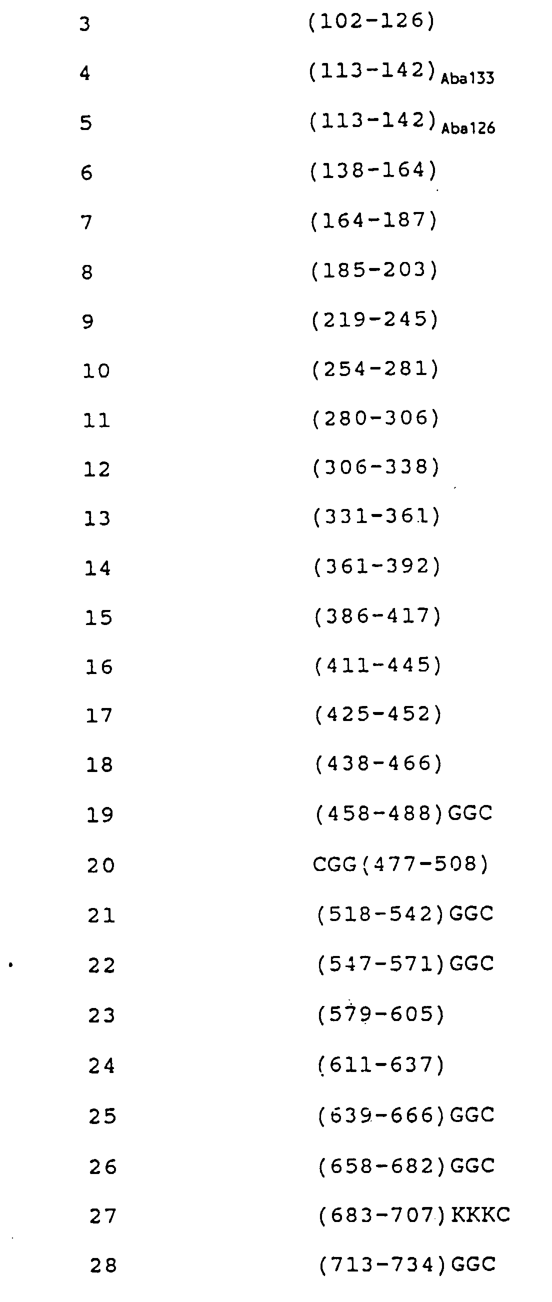

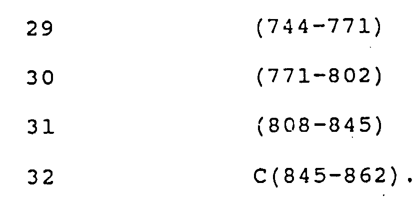

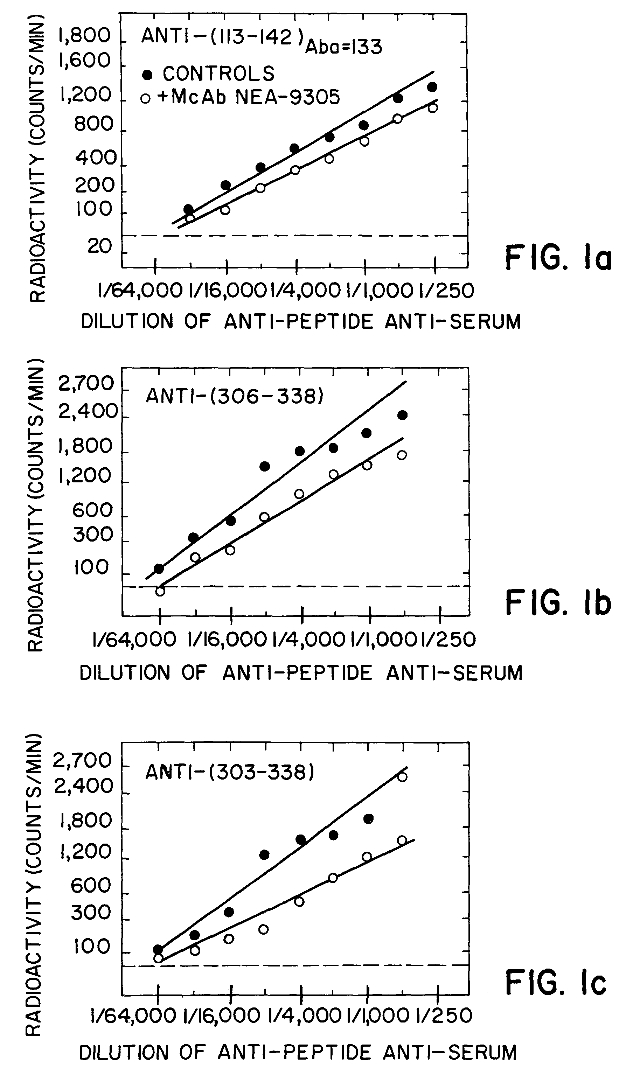

Results of these studies revealed that (a) as expected, mouse McAb to HIV-1 gp160 inhibited the attachment of rabbit anti-peptide antisera anti-(303-338) and anti-(306-338) (Figs. 1c and 1b); (b) among all other anti-peptide antisera studied, attachment of only anti-(113-142)_{Aba133} to gp120/gp160 was inhibited by the McAb (Fig. 1a).

Figs. 1a to 1c depict the effect of McAb NEA-9305 on the attachment of distinct anti-peptide antisera to HIV-1 gp160. Wells of polystyrene plates (Imulon II, Dynatech, Chantilly, VA) were coated with recombinant HIV-1 gp160 (500 ng/ml; MicroGeneSys Inc., West Haven, CT) and post-coated with bovine serum albumin (10 mg/ml) and gelatin (1 mg/ml). McAb NEA-9305 (Du pont, Boston, MA), was added to the wells at a final dilution of 1:500. After incubation for 30 minutes at 20°C, dilutions of anti-peptide antisera in 1% fetal bovine serum, 1% goat serum, pH 8 were added to the wells. After incubation overnight at 20°C, the attached rabbit antibodies were detected by ¹²⁵I-labeled goat rabbit anti-IgG. The wells were incubated with the radioactive antibody for two hours at 37°C, washed and counted for radioactivity. Radioactivity on the ordinate of Fig. 1 is plotted in a logit scale.

These results indicate that amino acid residues within the sequence (113-142) are spatially in the proximity of residues within the sequence (303-338) and suggest that residues from these non-adjacent portions of the primary amino acid sequence of gp120 comprise a functionally important conformational epitope which is recognized by virus-neutralizing antibodies. Since the monoclonal antibody did not compete with antibodies to the overlapping peptides (102-126) and (138-164), respectively, residues within the sequence (127-137) probably play a dominant role in this conformational epitope. These results agree with the observation that point mutations at position 135 affect the neutralizability of HIV-1 by antibodies (Willey et al, (1989), supra). In the peptides synthesized either C-residue at position 126 or at position 133 was replaced by γ -aminobutyric acid (Aba). Since antibodies to the peptide (113-142)_{Aba126} did not compete, the cysteine at position 126 is probably essential for this epitope, and cannot be replaced by aminobutyric acid, unlike the cysteine at residue 133. It is likely that a disulfide bond between residues 126 and 137 contributes to the conformational epitope.

### Example 2

Similar results as reported hereinabove were obtained when the inhibition of attachment to gp160 of rabbit anti-peptide antibodies by goat antiserum to peptide SP10 (residues 308-331) was studied.

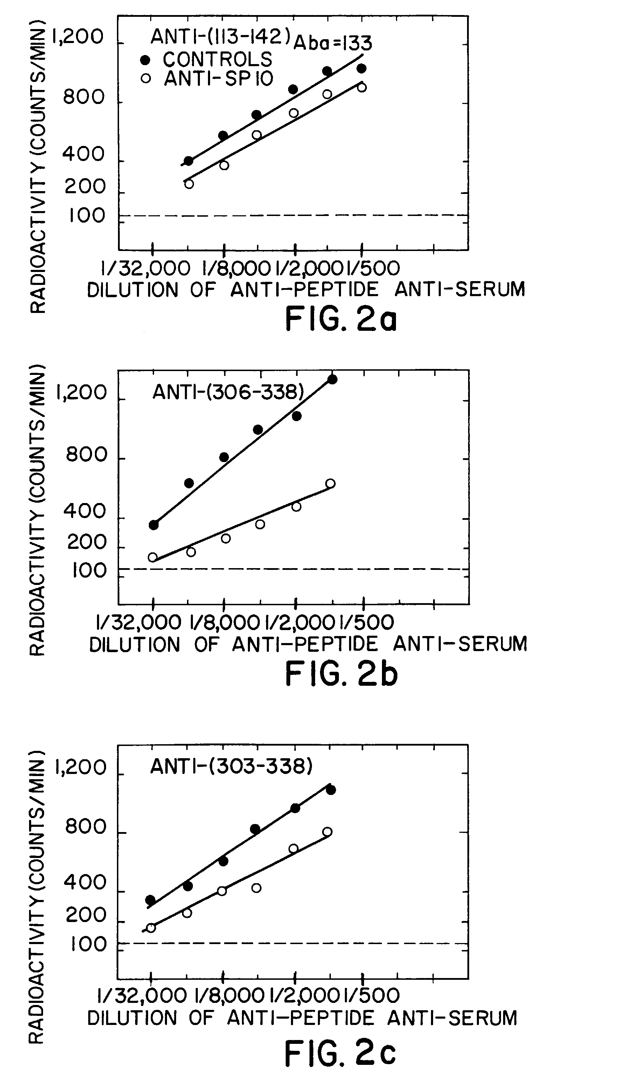

Figs. 2a to 2c depict the effect of goat anti-SP10 on the attachment of distinct rabbit anti-peptide antisera to HIV-1 gp160. Wells of polystyrene plates (Imunolon II, Dynatech, Chantilly, VA) were coated with recombinant HIV-1 gp160 (500 ng/ml; MicroGeneSys Inc., West Haven, CT) and post-coated with bovine serum albumin (10 mg/ml) and gelatin (1 mg/ml). Goat anti-SP10 (AIDS Research and Reference Reagent Program, National Institute of Allergy and Infectious Diseases, Bethesda, MD), was added to the wells at a final dilution of 1:100. In control experiments, goat antiserum to maleimidobenzoyl-N-hydroxysuccinimide ester (MBS)-derivatized tetanus toxoid was used. After incubation for 30 minutes at 20°C, dilutions of rabbit anti-peptide antisera in 1% fetal bovine serum, pH8 were added to the wells. After incubation overnight at 20°C, the attached rabbit antibodies were detected by ¹²⁵I-labeled goat rabbit anti-IgG. The wells were incubated with the radioactive antibody for two hours at 37°C, washed and counted for radioactivity. Radioactivity on the ordinates of Figs. 2a to 2c are plotted in a logit scale.

## Claims

1. A polypeptide comprising
(a) the amino acid sequence: 
                    126          137
EDIISLWDQSLKPCVKLTPLCVSLKCTDLK
113                    133      142
 of the envelope glycoprotein gp120 of the BH-10 isolate of the human immunodeficiency virus or an analogous amino acid sequence from a human immunideficiency virus gp120 isolate other than BH-10;
linked by an amino acid spacer sequence to:
(b) an amino acid sequence comprising 5 to 36 amino acids in the amino acid sequence: 
CTRPNNNTRKSIRIQRGPGRAFVTIGKIGNMRQAHC
303                                                    338
 of the envelope glycoprotein gp120 of the BH-10 isolate of the human immunodeficiency virus or an analogous amino acid sequence from a human immunodeficiency virus gp120 isolate other than BH-10;
said polypeptide not comprising more than 302 amino acids and said polypeptide not comprising a sequence identical with that of the entire natural gp120.

2. A polypeptide according to claim 1, comprising the amino acid sequence 113-338 of the envelope glycoprotein gp120 of the BH-10 isolate of the human immunodeficiency virus or an analogous amino acid sequence from a human immunodeficiency virus gp120 isolate other than BH-10.

3. A polypeptide comprising
(a) the amino acid sequence (126-137) of the envelope glycoprotein gp120 of the BH-10 isolate of the human immunodeficiency virus or an analogous amino acid sequence from a human immunodeficiency virus gp120 isolate other than BH-10;
linked by an amino acid spacer sequence to
(b) an amino acid sequence comprising 5 to 36 amino acids in the amino acid sequence: 
CTRPNNNTRKSIRIQRGPGRAFVTIGKIGNMRQAHC
303                                                                                    338
 of the envelope glycoprotein gp120 of the BH-10 isolate of the human immundeficiency virus or an analogous amino acid sequence from a human immunodeficiency virus gp120 isolate other than BH-10;
said polypeptide not comprising more than 302 amino acids and said polypeptide not comprising a sequence identical with that of the entire natural gp120.

4. A polypeptide according to any one of claims 1, 2 or 3, wherein γ-aminobutyric acid replaces C at position 133.

5. An anti-HIV-1 vaccine comprising an anti-HIV-1 effective amount of a polypeptide according to any one of claims 1, 2, 3 or 4 in admixture with a physiologically acceptable diluent.

6. A method of inducing the production of neutralizing antibodies to the human immunodeficiency virus comprising administering to an animal an anti-HIV-l-effective amount of a polypeptide according to any one of claims 1, 2, 3 or 4.

7. A composition comprising
(a) a polypeptide comprising the amino acid sequence: 
                    126         137
EDIISLWDQSLKPCVKLTPLCVSLKCTDLK
113                     133      142
 of the envelope glycoprotein gp120 of the BH-10 isolate of the human immunodeficiency virus or an analogous amino acid sequence from a human immunodeficiency virus gp120 isolate other than BH-10;
in admixture with
(b) a polypeptide comprising 5 to 36 amino acids in the amino acid sequence: 
CTRPNNNTRKSIRIQRGPGRAFVTIGKIGNMRQAHC
303                                338
 of the envelope glycoprotein gp120 of the BH-10 isolate of the human immunodeficiency virus or an analogous amino acid sequence from a human immunodeficiency virus gp120 isolate other than BH-10;
said composition not comprising any peptide comprising more than 302 amino acids and said composition not comprising a sequence identical with that of the entire natural gp120.

8. A composition comprising:
(a) a polypeptide comprising the amino acid sequence (126-137) of the envelope glycoprotein gp120 of the BH-10 isolate of the human immunodeficiency virus or an analogous amino acid sequence from a human immunodeficiency virus gp120 isolate other than BH-10;
in admixture with:
(b) a polypeptide comprising 5 to 36 amino acids in the amino acid sequence: 
CTRPNNNTRKSIRIQRGPGRAFVTIGKIGNMRQAHC
303                                338
 of the envelope glycoprotein gp120 of the BH-10 isolate of the human immunodeficiency virus or an analogous amino acid sequence from a human immunodeficiency virus gp120 isolate other than BH-10;
said composition not comprising any peptide comprising more than 302 amino acids and said composition not comprising a sequence identical with that of the entire natural gp120.

9. A composition according to claim 7 or 8, wherein in polypeptide (a) γ-aminobutyric acid replaces C at position 133.

10. A composition according to any one of claims 7, 8 or 9, wherein polypeptide (a) and polypeptide (b) are linked to a common carrier.

11. A composition according to any one of claims 7, 8 or 9, wherein polypeptide (a) is linked to a carrier and polypeptide (b) is linked to a separate carrier.

12. An anti-HIV-1 vaccine comprising an anti-HIV-1 effective amount of a composition according to any one of claims 7, 8, 9, 10 or 11 in admixture with a physiologically acceptable diluent.

13. A method of inducing the production of neutralizing antibodies to the human immunodeficiency virus comprising administering to an animal an anti-HIV-1 effective amount of a composition according to any one of claims 7, 8, 9, 10 or 11.

## Patentansprüche

1. Ein Polypeptid, umfassend
(a) die Aminosäuresequenz: 
126 137
EDIISLWDQSLKPCVKLTPLCVSLKCTDLK
113 113 142
 des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
verbunden durch eine Aminosäurespacersequenz mit:
(b) einer Aminosäuresequenz, umfassend 5 bis 36 Aminosäuren in der Aminosäuresequenz: 
CTRPNNNTRKSIRIQRGPGRAFVTIGKIGNMRQAHC
303                                                                                    338
 des Hüllenglycoproteins gp120 des BH-10-Isolats des menschlichen Immunschwäche-Virus oder eine analoge Aminosäuresequenz eines menschlichen Immunschwäche-Virus gp120-Isolats anderer Art als BH-10;
wobei dieses Polypeptid nicht mehr als 302 Aminosäuren umfaßt und dieses Polypeptid keine Sequenz umfaßt, die identisch mit der des gesamten natürlichen gp120 ist.

2. Ein Polypeptid nach Anspruch 1, umfassend die Aminosäuresequenz 113 bis 338 des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10.

3. Ein Polypeptid, umfassend
(a) die Aminosäuresequenz (126 bis 137) des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
verbunden durch eine Aminosäurespacersequenz mit
(b) einer Aminosäuresequenz, umfassend 5 bis 36 Aminosäuren in der Aminosäuresequenz: 
CTRPNNNTRKSIRIQRGPGRAFVTIGKIGNMRQAHC
303                                                    338
 des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
wobei dieses Polypeptid nicht mehr als 302 Aminosäuren umfaßt und dieses Polypeptid keine Sequenz umfaßt, die identisch mit der der gesamten natürlichen gp120 ist.

4. Ein Polypeptid, entsprechend einem der Ansprüche 1, 2 oder 3, worin γ-Aminobutansäure C in Position 133 ersetzt.

5. Ein Anti-HIV-1-Impfstoff, umfassend eine gegen HIV-1 wirksame Menge eines Polypeptids nach einem der Ansprüche 1, 2, 3 oder 4 in Mischung mit einem physiologisch verträglichen Verdünnungsmittel.

6. Ein Verfahren zur Anregung der Produktion von menschlichen Immunschwäche-Virus neutralisierenden Antikörpern, umfassend die Verabreichung einer gegen HIV-1 wirksamen Menge eines Polypeptids nach einem der Ansprüche 1, 2, 3 oder 4 an ein Tier.

7. Eine Zusammensetzung, umfassend
(a) ein Polypeptid, umfassend die Aminosäuresequenz: 
126 137
EDIISLWDQSLKPCVKLTPLCVSLKCTDLK
113 133 142
 des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
im Gemisch mit
(b) einem Polypeptid, umfassend 5 bis 36 Aminosäuren in der Aminosäuresequenz: 
CTRPNNNTRKSIRIQRGPGRAFVTIGKIGNMRQAHC
303                                                    338
 des Hüllenglycoproteins gp120 des BH-10-Isolats des menschlichen Immunschwäche-Virus oder einer analogen Aminosäuresequenz eines gp120-Isolats aus einem menschlichen Immunschwäche-Virus anderer Art als BH-10; wobei die Zusammensetzung keine Peptide mit mehr als 302 Aminosäuren umfaßt und diese Zusammensetzung keine Sequenz umfaßt, die identisch mit der der gesamten natürlichen gp120 ist.

8. Eine Zusammensetzung, umfassend:
(a) ein Polypeptid, umfassend die Aminosäuresequenz (126 - 137) des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
im Gemisch mit:
(b) einem Polypeptid, umfassend 5 bis 36 Aminosäuren in der Aminosäuresequenz: 
CTRPNNNTRKSIRIQRGPGRAFVTIGKIGNMRQAHC
303                                338
 des Hüllenglycoproteins gp120 des BH-10-Isolats des menschlichen Immunschwäche-Virus oder eine analoge Aminosäuresequenz eines gp120-Isolats aus einem menschlichen Immunschwäche-Virus anderer Art als BH-10;
wobei diese Zusammensetzung keine Peptide mit mehr als 302 Aminosäuren umfaßt und diese Zusammensetzung keine Sequenz umfaßt, die identisch mit der der gesamten natürlichen gp120 ist.

9. Eine Zusammensetzung nach einem der Ansprüche 7 oder 8, worin im Polypeptid (a) γ-Aminobutansäure C in Position 133 ersetzt.

10. Eine Zusammensetzung nach einem der Ansprüche 7, 8 oder 9, worin Polypeptid (a) und Polypeptid (b) mit einem gemeinsamen Träger verbunden sind.

11. Eine Zusammensetzung nach einem der Ansprüche 7, 8 oder 9, worin Polypeptid (a) mit einem Träger verbunden ist und Polypeptid (b) mit einem anderen Träger verbunden ist.

12. Ein Impfstoff gegen HIV-1, umfassend eine gegen HIV-1 wirksame Menge einer Zusammensetzung nach einem der Ansprüche 7, 8, 9, 10 oder 11 im Gemisch mit einem physiologisch verträglichen Verdünnungsmittel.

13. Ein Verfahren zur Anregung der Produktion von menschlichen Immunschwäche-Virus neutralisierenden Antikörpern, umfassend die Verabreichung einer gegen HIV-1-wirksamen Menge einer Zusammensetzung nach einem der Ansprüche 7, 8, 9, 10 oder 11 an ein Tier.

## Revendications

1. Polypeptide comprenant
(a) la séquence polypeptidique : 
126 137
EDIISLWDQSLKPCVKLTPLCVSLKCTDLK
113 133 142
 de la glycoprotéine d'enveloppe gp120 de l'isolat BH-10 du virus d'immunodéficience humain ou une séquence d'acides aminés analogue de gp 120 d'un isolat de virus d'immunodéficience humain, autre que le BH-10;
rattaché, par une séquence d'acides aminés d'espacement, à
(b) une séquence d'acides aminés comprenant 5 à 32 acides aminés choisie dans la séquence d'acides aminés : 
CTRPNNNTRKSIRIQRGPGRAFVTIGKIGNMRQAHC
303                                338
 de la glycoprotéine d'enveloppe gp120 de l'isolat BH-10 du virus d'immunodéficience humain ou une séquence d'acides aminés analogue de gp120 d'un isolat de virus d'immunodéficience humain, autre que le BH-10;
ledit polypeptide ne comprenant pas plus de 302 acides aminés et ledit polypeptide ne comprenant pas une séquence identique avec celle de la gp120 naturelle et complète.

2. Polypeptide selon la revendication 1 comprenant la séquence d'acides aminés 113-338 de la glycoprotéine gp120 de l'isolat BH-10 du virus d'immunodéficience humain ou une séquence analogue de gp120 d'un isolat de virus d'immunodéficience humain autre que le BH-10.

3. Polypeptide comprenant
(a) la séquence d'acides aminés (126-137) de la glycoprotéine d'enveloppe gp120 de l'isolat BH-10 du virus d'immunodéficience humain ou une séquence d'acides aminés analogue de la gp120 d'un isolat de virus d'immunodéficience humain, autre que le BH-10;
rattaché, par une séquence d'acides aminés d'espacement, à
(b) une séquence d'acides aminés comprenant de 5 à 36 acides aminés choisie dans la séquence d'acides aminés : 
CTRPNNNTRKSIRIQRGPGRAFVTIGKIGNMRQAHC
303                                                                                    338
 de la glycoprotéine d'enveloppe gp120 de l'isolat BH-10 du virus d'immunodéficience humain ou une séquence d'acides aminés analogue à la gp120 d'un isolat de virus d'immunodéficience humain, autre que le BH-10;
ledit polypeptide ne comprenant pas plus de 302 acides aminés et ledit polypeptide ne comprenant pas une séquence identique avec celle de la gp120 naturelle et complète.

4. Un polypeptide selon n'importe laquelle des revendications 1, 2 ou 3, dans lequel l'acide -aminobutyrique remplace C à la position 133.

5. Vaccin anti-HIV-1 comprenant une quantité efficace contre le HIV-1 d'un polypeptide selon n'importe laquelle des revendications 1, 2, 3 ou 4 en mélange avec un diluant physiologiquement acceptable.

6. Procédé pour induire la production d'anti-corps neutralisants contre le virus d'immunodéficience humain comprenant l'étape d'administration à un animal d'une quantité efficace contre le HIV-1 d'un polypeptide selon n'importe laquelle des revendications 1, 2, 3 ou 4.

7. Une composition comprenant
(a) un polypeptide comprenant la séquence d'acides aminés 
                 126        137
EDIISLWDQSLKPCVKLTPLCVSLKCTDLK
113                 133      142
 de la glycoprotéine d'enveloppe gp120 de l'isolat BH-10 du virus d'immunodéficience humain ou une séquence d'acides aminés analogue de gp120 d'un isolat du virus d'immunodéficience humain, autre que le BH-10;
en mélange avec
(b) un polypeptide comprenant de 5 à 36 acides aminés choisie dans la séquence d'acides aminés 
CTRPNNNTRKSIRIQRGPGRAFVTIGKIGNMRQAHC
303                                                    338
 de la glycoprotéine d'enveloppe gp120 de l'isolat BH-10 du virus d'immunodéficience humain ou une séquence d'acides aminés analogue de gp120 d'un isolat du virus d'immunodéficience humain, autre que le BH-10;
ledit polypeptide ne comprenant pas plus de 302 acides aminés et ledit polypeptide ne comprenant pas une séquence identique avec celle de la gp120 naturelle et complète.

8. Une composition comprenant
(a) un polypeptide comprenant la séquence d'acides aminés (126-137) de la glycoprotéine d'enveloppe gp120 de l'isolat BH-10 du virus d'immunodéficience humain ou une séquence d'acides aminés analogue de la gp 120 d'un isolat de virus d'immunodéficience humain, autre que le BH-10;
en mélange avec:
(b) un polypeptide comprenant de 5 à 36 acides aminés choisie dans la séquence d'acides aminés : 
CTRPNNNTRKSIRIQRGPGRAFVTIGKIGNMRQAHC
303                                338
 de la glycoprotéine d'enveloppe gp120 de l'isolat BH-10 du virus d'immunodéficience humain ou une séquence d'acides aminés analogue de gp120 d'un isolat de virus d'immunodéficience humain, autre que le BH-10;
ledit polypeptide ne comprenant pas plus de 302 acides aminés et ledit polypeptide ne comprenant pas une séquence identique avec celle de la gp120 naturelle et complète.

9. Une composition selon la revendication 7 ou 8 dans laquelle dans le polypeptide (a) l'acide -aminobutyrique remplace C à la position 133.

10. Une composition selon n'importe laquelle des revendications 7, 8 ou 9, dans laquelle le polypeptide (a) et le polypeptide (b) sont reliés par un substrat porteur commun.

11. Composition selon n'importe laquelle des revendications 7, 8 ou 9 dans laquelle le polypeptide (a) est rattaché à un substrat porteur et le polypeptide (b) est rattaché à un substrat porteur séparé.

12. Vaccin anti-HIV-1 comprenant une quantité efficace contre le HIV-1 d'une composition selon n'importe laquelle des revendications 7, 8, 9, 10 ou 11 en mélange avec un diluant physiologiquement acceptable.

13. Procédé pour induire la production d'anticorps neutralisants contre le virus d'immunodéficience humain comprenant l'étape d'administration à un animal d'une quantité efficace contre le HIV-1 d'une composition suivant n'importe laquelle des revendications 7, 8, 9, 10 ou 11.
